# DEMANDE DE BREVET EUROPEEN

(11) **EP 4 803 085 A1**
(43) Date de publication de la demande: **09.09.2026**
(21) Numéro de dépôt: 25161426.9
(22) Date de dépôt: 03.03.2025
(51) Int. Cl.: A61K 31/683, A61K 31/685, A61K 31/688, A61P 17/00, A61P 17/06, A61P 17/12, A61K 36/00

(54) **PHOSPHOLIPIDES ESTÉRIFIÉS PAR DES ACIDES GRAS POLYINSATURÉS OMÉGA-3 DHA DESTINÉS À ÊTRE UTILISÉS DANS LE TRAITEMENT DU PSORIASIS**

(71) Demandeur: Dupont, Paul, 31770 Colomiers Occitanie (FR); SC Dicophar, 31770 Colomiers (FR)
(72) Inventeur: DUPONT, Paul, 31770 colomiers (FR)

(57) **Abrégé**

La présente invention se rapporte à l'emploi d'un phospholipide estérifié par des acides gras polyinsaturés de type oméga-3 DHA ou d'un extrait lipidique riche en oméga-3 DHA sous forme de phospholipides pour la préparation d'un complément alimentaire destiné au maintien d'une peau normale ou pour la préparation d'une composition pharmaceutique utile dans la prévention et le traitement thérapeutique des dermatoses récentes ou anciennes, notamment du psoriasis et de l'eczéma. Les compositions thérapeutiques comprenant un phospholipide estérifié par des acides gras polyinsaturés de type oméga-3 DHA ou un extrait lipidique riche en oméga-3 DHA sous forme de phospholipides sont également objet de la présente invention. Selon une caractéristique avantageuse de la présente invention, les phospholipides sont estérifiés par des acides gras polyinsarurés du type oméga-3, en particulier par l'acide docosahexanoïque (DHA). Ces phospholipides riches en oméga-3 DHA pourront être un extrait animal ou végétal choisi de préférence parmi les poissons, les crevettes, le krill, le zooplancton, les algues, le phytoplancton, ou extrait d'oeuf d'origine animale ou bien encore d'un mélange de ceux-ci. Ils pourront être aussi obtenus par synthèse enzymatique de phospholipides structurés riches en DHA.

## Description

La présente invention se rapporte à l'emploi d'un phospholipide estérifié par des acides gras polyinsaturés de type oméga-3 DHA ou d'un extrait lipidique riche en oméga-3 DHA sous forme de phospholipides pour la préparation d'un complément alimentaire destiné au maintien d'une peau normale ou pour la préparation d'une composition pharmaceutique utile dans la prévention et le traitement thérapeutique des dermatoses récentes ou anciennes, notamment du psoriasis et de l'eczéma.

Les formations squameuses et/ou kératosiques anormales du derme sont une des manifestations de l'état de la peau qui se rencontre dans de nombreuses dermatoses, qu'elles soient apparues de façon récentes ou installées de longue date, et en particulier dans les différentes formes du psoriasis et d'eczéma.

Le psoriasis est une dermatose érythémo-squameuse d'évolution chronique, caractérisée par des plaques rouges recouvertes d'épaisses squames blanches, qui atteint environ 2% de la population. Avec plus de 60 000 nouveaux cas chaque année, c'est une des affections dermatologiques les plus fréquentes. Elle se manifeste par des démangeaisons douloureuses et des plaies pouvant atteindre plus de 10% du corps.

Dans ses formes habituelles, sa localisation est très caractéristique : coudes, et bord cubital des avant-bras, genoux, région-lombo-sacrée, cuir chevelu, ongles.
Des formes particulières, qualifiées de psoriasis graves, sont également connues, telles que le psoriasis érythrodermique, le rhumatisme psoriasique ou le psoriasis pustuleux.

Environ 1,5% des psoriasis débutent chez l'enfant avant l'âge de 10 ans et 35 % avant 20 ans. Cette anomalie du renouvellement de l'épiderme est donc une affection sérieuse représentant un problème de santé publique important.

La cause du psoriasis est pour l'instant inconnue mais on considère que les facteurs impliqués sont multiples. Un facteur génétique a pu être mis en évidence, avec 30% de psoriasis familiaux, ainsi que des facteurs environnementaux. Les facteurs psychologiques peuvent fréquemment déclencher des poussées. Les traumatismes cutanés tels que griffures, vaccinations, chirurgie, sont parfois le siège d'une efflorescence de lésions psoriasiques nommé phénomène de Koebner.

L'alcool et le tabac sont des facteurs d'aggravation et de résistance thérapeutique. L'évolution de la maladie est chronique et se fait par poussées généralement imprévisibles, entrecoupées de rémissions pendant lesquelles les lésions sont minimes. Même en dehors des formes graves, et bien que l'état général du malade ne soit pas altéré dans les formes habituelles, le psoriasis est une maladie qui peut perturber profondément la qualité de vie lorsque les lésions sont visibles ou gênantes pour un travail manuel, conséquences qui sont souvent sous-estimées par le médecin.

A l'heure actuelle, les médecins disposent de traitements symptomatiques aux résultats transitoires, mais pas encore de médicaments curatifs. Les traitements locaux, sous forme de pommade ou de crème, sont des lotions à base de cortisone et les dérivés de la vitamine D, ces deux produits étant souvent combinés. Les rétinoïdes (dérivés de la vitamine A) peuvent également être utilisés. Ils sont très utiles mais ne peuvent être correctement employés que si le psoriasis n'est pas trop étendu ou évolue peu. S'il dépasse 10% de la surface corporelle, près de la moitié des patients arrêtent de suivre ces traitements contraignants au bout d'un mois, faute de temps. En outre les dérivés de la vitamine D ont un potentiel irritant, et surtout les corticoïdes peuvent provoquer une atrophie cutanée et entraîner des rebonds de la maladie.

Les séances de photothérapie sont efficaces chez un grand nombre de patients. Elles consistent en une irradiation en cabine médicale par des rayons UVB ou UVA, en association avec des médicaments photosensibilisants. Les cures sont contraignantes, car les patients doivent se soumettre à trois séances hebdomadaires de deux heures pendant 8 à 10 semaines. Cette technique présente surtout l'inconvénient majeur d'un risque d'induction à long terme de cancers cutanés, ainsi que d'un vieillissement accéléré de la peau. Il est donc indispensable de la réaliser selon des règles très strictes.

Pour les patients qui n'ont pas été soulagés par les traitements locaux ou par la photothérapie, soit 30 % à 40% des malades, des traitements généraux sont proposés. Un de ces traitements repose sur la prise de rétinoïdes par voie orale, le plus souvent en association avec la photothérapie ou bien avec la prise de méthotréxate qui est le médicament de référence. Ce dernier ayant des effets secondaires indésirables, notamment hématologiques et hépatiques, sa prescription nécessite une surveillance étroite. La ciclosporine (Néoral ^{®}) est également efficace, mais du fait de sa toxicité rénale, elle ne peut pas être préconisée pour des traitements prolongés. On comprend donc que les traitements préconisés jusqu'à présent ont tous des limitations importantes quant à leur prescription et à leur tolérance, et que leur efficacité reste incertaine et provisoire dans de nombreux cas. Plus récemment on a recours à la biothérapie par les anticorps monoclonaux. Ces anticorps visent à bloquer le phénomène psoriasique. On réserve ce traitement aux psoriasis qui résistent à au moins deux des traitements classiques, ou qui évoluent en arthrite psoriasique. Ces anticorps monoclonaux vont cibler spécifiquement un médiateur de l'inflammation à l'origine de la maladie. Pour deux tiers des patients, la biothérapie offre une réduction de 75 % des symptômes. Mais en raison de ses effets notamment sur la diminution des défenses immunitaires augmentant le risque d'infections, elle est réservée aux formes les plus invalidantes. D'autant que dans certains cas cette thérapeutique peut entraîner des rebonds de la maladie pendant ou après l'arrêt du traitement.

Dans cette situation, le présent inventeur a conduit des essais en vue d'identifier une substance susceptible d'être utilisée dans le traitement du psoriasis et plus généralement dans le traitement des dermatoses et de toute affection cutanée se manifestant ou provoquée par des formations kératosiques et/ou squameuses anormales du derme, qu'elles soient aiguës ou chroniques. De façon surprenante et inattendue, il avait déjà découvert par le passé que l'administration d'une composition à base de lécithine marine à un patient souffrant d'une telle affection conduit à d'excellents résultats, ce qui a abouti à divers brevets concernant cette précédente invention.

Cependant, les résultats obtenus par la suite avec diverses compositions de lécithines dites marines n'ont pas été toujours concluants. Les sources de lécithines étaient en effet variables. Divers types de lécithines étaient utilisés soit à base de peau de poisson, d'œufs de poisson, d'huile de krill. Cela laissait entendre que seuls certains phospholipides de ces compositions de lécithine pouvaient avoir un effet thérapeutique.

La présente invention est le fruit de la poursuite de la recherche et plus particulièrement d'une étude comparative portant sur l'utilisation de différents types de lécithines. Elle est le fruit d'un questionnement portant sur l'intérêt de différents types de phospholipides estérifiés par divers acides gras polyinsaturés de type oméga-3 DHA et/ou EPA qui puissent être utiles en dermatologie. On connaît déjà les bénéfices de ce type de phospholipides dans les pathologies neurologiques ou cardio-vasculaires. Mais aucune découverte n'a été faite dans le domaine de la peau humaine. La recherche s'est donc poursuivie pour évaluer l'utilité des phospholipides tels que ceux contenus dans des lécithines marines ou huiles marines.

De manière générale, 5 phospholipides sont regroupés dans les lécithines de divers organismes vivants. Mais ils ne sont pas tous estérifiés par les mêmes acides gras essentiels. Ceux contenus dans les végétaux le sont en général par des acides gras oméga-6, tandis que ceux des organismes animaux le sont par des acides gras oméga-3. Dans la présente recherche nous avons choisi de comparer les phospholipides contenus dans de l'huile de krill qui sont majoritairement estérifiés par des acides gras oméga-3 EPA avec les phospholipides extraits d'œufs de hareng majoritairement estérifiés par des acides gras oméga-3 DHA. Il est alors apparu de manière surprenante que le seul produit véritablement actif sur la peau, notamment en cas de psoriasis et d'eczéma, est une variété de phospholipides riches en acides gras polyinsaturés oméga-3 DHA (oméga-3 DHA phospholipides) et non pas en oméga-3 EPA (oméga-3 EPA phospholipides).

Une étude comparative a permis ainsi de découvrir que seuls les phospholipides riches en acides gras polyinsaturés oméga-3 DHA (oméga-3 DHA phospholipides) pouvait améliorer ces pathologies. Elle a été menée chez plusieurs personnes pour savoir lequel des phospholipides est efficace parmi ceux qui se trouvent dans les deux types de lécithine marine. Un premier groupe a consommé de l'huile de krill qui contient surtout des phospholipides estérifiés par des acides gras polyinsaturés de type oméga-3 EPA et l'autre groupe a reçu un extrait d'œufs de hareng qui contient des phospholipides estérifiés par des acides gras polyinsaturés de type oméga-3 DHA. Les résultats après 4 mois ont montré qu'à dose équivalente de lécithine, ceux supplémentés par des phospholipides estérifiés par des acides gras polyinsaturés de type oméga-3 EPA avaient tendance à voir leur psoriasis stagner voire à s'aggraver, tandis que ceux recevant les phospholipides estérifiés par des acides gras polyinsaturés de type oméga-3 DHA avaient une amélioration nette de leur psoriasis.

Cette découverte inattendue a donc montré que seules des compositions les plus riches en phospholipides estérifiés par des acides gras polyinsaturés de type oméga-3 DHA permettaient de préparer un tel médicament. Avec parmi ces phopholipides une majorité de phosphatidylcholine. L'invention réside donc dans l'emploi de phospholipides estérifiés par des acides gras polyinsaturés de type oméga-3 DHA ou d'oméga-3 DHA sous forme de phospholipide, notamment la phosphatidylcholine pour la préparation d'un médicament efficace dans la prévention et le traitement des dermatoses et autres affections récentes ou chroniques du derme, qui ne présente pas d'effet secondaire indésirable et dont la forme soit commode d'emploi pour le patient. Les compositions thérapeutiques sous la forme de médicament ou de compléments alimentaires résultant de la présente découverte sont également objet de la présente invention.

C'est pourquoi la présente invention a pour objectif de proposer une composition utile pour le traitement curatif des états provoqués ou se manifestant par des formations kératosiques et/ou squameuses anormales du derme, telles que celles qui se rencontrent dans les dermatoses récentes ou chroniques, et en particulier dans les différentes formes du psoriasis. Une action prophylactique est également recherchée.

Les phospholipides estérifiés par des acides gras polyinsaturés de type oméga-3 DHA sont des phospholipides porteurs d'acides gras oméga 3 DHA. Ils sont considérés au même titre que les phospholipides estérifiés par des acides gras polyinsaturés de type oméga-3 EPA comme des substances indispensables au bon fonctionnement du système cardio-vasculaire, du cerveau, du système nerveux, du foie, de l'œil et de nombreux autres organes.

Selon un usage commun, les phospholipides estérifiés par des acides gras polyinsaturés de type oméga-3 DHA sont souvent intégrés à des complexes composés principalement de plusieurs phospholipides naturels (ou phosphoglycérides), à savoir essentiellement la phosphatidylcholine, le phosphatidylinositol, la phosphatidyléthanolamine, la phosphatidylsérine auxquels s'ajoute la sphingomyéline et on les regroupe en général sous le terme de lécithine.

Dans le règne animal et chez les êtres humains, ces phosphoglycérides peuvent en outre être estérifiés sur la fonction alcool primaire libre par des acides gras, saturés ou insaturés. Ces acides gras peuvent notamment être des acides gras polyinsaturés du type oméga-3, en particulier l'acide docosahexanoïque (DHA), l'acide eicosapentanoïque (EPA), l'acide docosapentanoïque (DPA). Il peut aussi s'agir d'un acide gras de type alkyl-glycérol. Le profil de répartition de ces différents phospholipides et de leurs dérivés estérifiés est variable d'un organisme à l'autre.

L'acide docosahexaénoïque ou DHA est un acide gras polyinsaturé oméga-3 de formule C₂₂H₃₂O₂. Les micro-algues ainsi que les bactéries qui synthétisent le DHA sont le point de départ de ce nutriment pour la chaîne alimentaire marine. On le retrouve également dans les œufs de poissons où il est principalement sous forme d'oméga-3 DHA phosphatidylcholine. Dans l'huile de krill par contre, l'oméga-3 estérifié sur les phospholipides est majoritairement l'EPA.

Les phospholipides estérifiés par des acides gras polyinsaturés de type oméga-3 DHA objets de la présente invention peuvent être extraits des œufs de poisson, des algues d'origine marine ou d'eau douce, des œufs de poules dont l'alimentation a été supplémentée en oméga-3 DHA et des lécithines de poissons. Les méthodes d'extraction sont connues en soi. Les phospholipides sont usuellement employés en alimentation animale et comme complément nutritionnel en diététique humaine.

Cependant leur action en tant que médicament sur les affections cutanée était jusqu'à présent totalement inconnue, en dehors de l'action de la lécithine marine qui avait déjà été mise en évidence pour la première fois par le présent inventeur (brevet Européen EP1871399B1). Les lécithines marines, c'est-à-dire extraites des organismes marins, se sont avérées particulièrement intéressantes mais la discordance apparue entre les différentes types de lécithine n'avait pas permis de préciser les phospholipides véritablement actifs. La présente invention permet donc de pallier ce manque puisqu'elle met en évidence le fait que ce sont les phospholipides estérifiés par des acides gras polyinsaturés de type oméga-3 DHA qui sont utiles pour la mise en œuvre de la présente invention.

Un premier objet de l'invention est donc l'utilisation des phospholipides estérifiés par des acides gras polyinsaturés de type oméga-3 DHA dans une composition destinée à la fabrication d'un médicament utile dans la prévention et le traitement des états provoqués ou se manifestant par des formations squameuses et/ou kératosiques anormales du derme.

En particulier, ladite utilisation s'applique aux états choisis parmi le psoriasis habituel en gouttes, nummulaires, en plaques, le psoriasis érythrodermique, le psoriasis arthropathique, le psoriasis pustuleux, le psoriasis de l'enfant, le parapsoriasis, la pulpite digitale psoriasique, les dermatoses aiguës ou chroniques telles que les dermatoses ichtyosiques et kératosiques dont la kératose palmo-plantaire ainsi que l'eczéma.

Le second objet de l'invention est l'utilisation des phospholipides estérifiés par des acides gras polyinsaturés de type oméga-3 DHA dans une composition destinée à la fabrication d'un médicament utile dans la prévention et le traitement des états provoqués ou se manifestant par des formations érythémateuses squameuses et inflammatoires du type d'eczéma.

Le troisième objet de l'invention est l'utilisation des phospholipides estérifiés par des acides gras polyinsaturés de type oméga-3 DHA dans la composition d'un complément alimentaire destiné au maintien d'une peau normale.

Selon un aspect particulier de l'invention, l'extrait comprend un complexe de phospholipides majoritairement riche en phospholipides estérifiés par des acides gras polyinsaturés de type oméga-3 DHA dans une quantité de 10% à 50% en poids, dans une phase grasse. De préférence, les phospholipides estérifiés par des acides gras polyinsaturés de type oméga-3 DHA sont présents à hauteur de 30% en poids dans l'extrait. L'extrait se présente comme une huile liquide pouvant aller du jaune au brun selon son origine, et prenant une teinte brune orangée translucide lorsqu'elle est extraite des œufs de poisson. Elle est sensible à l'oxydation. Son acidité oléique est de 0,1%, son indice d'iode de 170 et son indice de peroxyde maximum de 5 meq/kg. Elle ne présente aucune toxicité aux doses d'emploi, chez l'animal comme chez l'humain.

Selon un aspect préféré de la présente invention, lesdits phospholipides sont estérifiés par les acides gras du type oméga-3 DHA, dans les proportions exprimées en poids rapporté au poids total desdits acides gras:
- par l'acide docosahexanoïque (DHA) à raison de 15% à 85%,
- par l'acide eicosapentanoïque (EPA), à raison de 5% à 35%,
- par l'acide docisapentanoïque (DPA), à raison de 0,5% à 5%,
- l'acide gras de type alkyl-glycérol à raison de 5% à 30%.
De manière générale, les phospholipides sont majoritairement estérifiés par le DHA.

Selon une caractéristique intéressante, dans la composition thérapeutique selon l'invention, les phospholipides estérifiés par des acides gras polyinsaturés de type oméga-3 DHA comprennent les phospholipides suivants, en poids rapporté au poids total des phospholipides :
- de 30% à 85% de phosphatidylcholine, de préférence 50%,
- de 10% à 30% de phosphatidylinositol, de préférence 25%,
- de 5% à 30% de phosphatidyléthanolamine, de préférence 10%,
- de 5% à 20% de phosphatidylsérine, de préférence 10%, et
- de 5% à 20% de sphingomyéline, de préférence 5%.

De préférence, les phospholipides estérifiés par des acides gras polyinsaturés de type oméga-3 DHA comprennent les phospholipides suivants, en poids rapporté au poids total des phospholipides :
- 50% de phosphatidylcholine,
- 25% de phosphatidylinositol,
- 10% de phosphatidyléthanolamine,
- 10% de phosphatidylsérine, et
- 5% de sphingomyéline

Selon une caractéristique avantageuse de la présente invention, lesdits phospholipides peuvent être synthétisés par une estérification des acides gras polyinsaturés du type oméga-3 DHA, par synthèse enzymatique de phospholipides structurés enrichis en oméga-3 DHA. En effet, une fonction alcool primaire est présente dans les phospholipides cités plus haut, qui peut donc participer à une réaction d'estérification avec un acide, les acides gras polyinsaturés du type oméga-3 DHA étant préférés.

Selon une caractéristique intéressante de l'utilisation conforme à l'invention, l'extrait peut être produit par un organisme marin ou végétal choisi parmi les poissons, les crevettes, le zooplancton, les algues marines ou d'eau douce, le krill, le phytoplancton ou d'un mélange de ceux-ci. Son intérêt particulier réside dans le fait que ses phospholipides, notamment la phosphatidylcholine, sont naturellement estérifiés par des acides gras de type oméga-3, et majoritairement par l'oméga-3 DHA. Par extension on peut envisager d'utiliser des lécithines d'œufs animaux, notamment de poules supplémentées en oméga-3 DHA.

Selon un mode préféré de réalisation de l'invention les phospholipides estérifiés par des acides gras polyinsaturés de type oméga-3 DHA peuvent être extraits des œufs de poisson des mers froides, ladite phase grasse étant associée à une huile de poisson, de préférence une huile de poisson également riche en oméga 3-DHA comme par exemple le hareng. De manière alternative, l'extrait produit pour l'utilisation selon l'invention peut également se présenter sous forme purifiée en poudre ou incorporé dans une teinture hydro-alcoolique.

Finalement, ledit médicament peut avantageusement être constitué de tout extrait lipidique contenant des phospholipides estérifiés par des acides gras polyinsaturés de type oméga-3 DHA dans une quantité de 5% à 100%, de préférence de 10% à 30% en poids. Le médicament peut aussi être constitué d'extraction sélective de ces phospholipides estérifiés par des acides gras polyinsaturés de type oméga-3 DHA purs. De sorte que le produit puisse être utilisé selon une posologie permettant l'apport d'une dose efficace, se situant entre 400 mg et 1000 mg d'oméga-3 DHA phospholipides par jour. La forme orale est préférée, car la prise induit très peu de contrainte, ce qui permet une bonne tolérance des patients pour des cures d'une durée de plusieurs mois.

Le médicament préparé à l'aide de la composition comprenant les oméga-3 DHA phospholipides peut aussi se trouver sous une forme administrable par voie rectale ou par voie lymphatique directe. L'extrait huileux, comme les formes purifiées, peuvent être conditionnés en globules ou liposomes. Il est également possible de présenter la composition selon l'invention sous toute forme telle que capsules, comprimés, suppositoires, ovules, crème, lotion, lait, pommade, gel ou poudre.

Si nécessaire, le médicament peut comprendre en outre un excipient convenant à une administration par voie orale, par voie rectale ou par voie lymphatique directe. Ce sera le cas notamment des compositions appliquées sur la peau. Un tel excipient peut être judicieusement choisi par l'homme du métier parmi des excipients d'usage thérapeutique neutres vis-à-vis des actifs, en fonction de la voie d'administration retenue et de la consistance finale souhaitée. Par exemple, on peut utiliser l'eau, l'alcool, la glycérine, le propylène glycol, le butylène glycol, les diglycérols éthoxylés ou propoxylés.

Les phospholipides estérifiés par des acides gras polyinsaturés de type oméga-3 DHA utilisés conformément à la présente invention peuvent bien entendu être accompagnés d'autres composants tels que des additifs ou des supports de vectorisation, selon les techniques connues de l'homme de l'art. Les additifs sont par exemple ceux nécessaires à la formulation convenable du médicament contenant
ces phospholipides tels que des épaississants, des tensio-actifs, des anti-oxydants, des colorants, des conservateurs, des parfums. La vectorisation pourra être réalisée par solubilisation dans des liposomes, adsorption sur des polymères organiques poudreux, sur des supports minéraux comme le talc ou les bentonites, la maltodextrine, la gomme arabique, l'amidon ou à l'aide de tout autre vecteur pharmaceutiquement acceptable.

L'objet de la présente invention, ainsi que ses modes de mise en œuvre possibles et les avantages qui y sont liés, apparaîtront plus clairement à la lecture des études que nous avons menées. Celles-ci à titre informatif permettent de vérifier le bien fondé de la présente invention. Les résultats suivants sont donnés à titre illustratif.

Le but de ces études était de découvrir quel type de phospholipide permet d'améliorer le psoriasis. Et pour cela deux études ont été réalisées avec l'administration des extraits lipidiques riches en phospholipides chez des personnes atteintes de psoriasis qui ne prenaient aucun autre traitement. L'étude numéro 1 consistait à comparer deux types d'extraits lipidiques de deux origines choisis par le fait qu'ils contenaient des composés de phospholipides différents. L'étude numéro 2 consistait à confirmer l'efficacité de l'une de ces deux variétés de composés de phospholipides sur le psoriasis.

### Étude numéro 1 : N1

Il s'agit d'une étude comparative randomisée entre deux composés de phospholipides. 40 personnes ont participé à l'étude. Après tirage au sort aléatoire les participants ont été divisés en deux groupes. Chacune des 20 personnes des deux groupes prenaient 2 capsules matin et soir de l'un ou l'autre des composés de phospholipides.

Caractérisation des extraits de phospholipides utilisés dans l'étude : L'étude a porté sur deux composés lipidiques riches en phospholipides. Les deux composés choisis étaient sous forme de capsules d'huile marine riches en phospholipides. La caractéristique particulière permettant la comparaison est leur teneur équivalente en phospholipides ; la différence réside dans la qualité des oméga-3 de ces phospholipides : il s'agissait en effet de phospholipides estérifiés par des acides gras polyinsaturés de type oméga-3 DHA dans le premier groupe et de phospholipides estérifiés par des acides gras polyinsaturés de type oméga-3 EPA dans le deuxième groupe.

L'un des deux composés de phospholipides de l'étude contient donc des phospholipides majoritairement riches en oméga-3 DHA phospholipides, notamment en phosphatidylcholine portant des acides gras oméga-3 DHA (c'est le groupe nommé Pc-DHA) tandis que l'autre composé est à base des phospholipides majoritairement riches en oméga-3 EPA phospholipides, avec la phosphatidylcholine estérifiée par des acides gras oméga-3 EPA (groupe nommé Pc-EPA). Ainsi il a été possible de comparer l'action de deux types de phospholipides : Pc-EPA et Pc DHA.

De manière plus descriptive le groupe nommé Pc-DHA a pris le composé lipidique Pc-DHA: Cet extrait contient 32 % de phospholipides d'œuf de Hareng sauvage des mers froides. Ces phospholipides sont composés de : 87 % de phosphatidylcholine. Les phospholipides sont estérifiés par l'acide docosahexanoïque (DHA) à raison de 75%, et à l'acide eicosapentanoïque (EPA) à raison de 14 %. L'acide docosapentanoïque (DPA) est présent à un taux d'environ 1%, et l'acide gras de type alkyl-glycérol à un taux d'environ 5 %. La phase grasse est constituée d'huile de poisson riche à 47% en OMEGA 3 selon un rapport DHA /EPA de 7/3. L'extrait est conditionné en doses de 500 mg sous forme de capsules de gélatine selon les techniques connues de la galénique. Aucun additif ni excipient n'est ajouté. Chaque capsule contient une dose utile de 160 mg de phospholipides et il est conseillé de prendre 2 capsules matin et soir.

Les personnes de ce groupe nommé Pc DHA qui prenaient cet extrait ont donc reçu au total chaque jour 640 mg de phospholipides majoritairement sous forme d'oméga-3 DHA phospholipides (77 %) : soit une prise d'environ 480 mg d'oméga-3 DHA phospholipides par jour et seulement 89 mg d'oméga-3 EPA phospholipides. La phase grasse est constituée d'un rapport DHA /EPA de 7/3.

Le deuxième groupe Pc EPA a reçu un composé lipidique nommé Pc EPA : Ce composé contient de l'huile de krill avec 40 % de phospholipides. Ces phospholipides sont composés de : 87 % de phosphatidylcholine. Les phospholipides sont estérifiés par l'acide eicosapentanoïque (EPA) à raison de 55 % et à l'acide docosahexanoïque (DHA) à raison de 25 %. L'acide docosapentanoïque (DPA) est présent à un taux d'environ 1%, et l'acide gras de type alkyl-glycérol à un taux d'environ 5 %. La phase grasse est constituée d'huile de krill selon un rapport DHA /EPA de 8/17. Le composé est conditionné en doses de 500 mg sous forme de capsules de gélatine selon les techniques connues de la galénique. Aucun additif ni excipient n'est ajouté. Chaque capsule contient une dose utile de 168 mg de phospholipides et il est conseillé de prendre 2 capsules matin et soir.

Les personnes de ce groupe nommé Pc EPA qui prend ce composé ont donc reçu au total chaque jour 672 mg de phospholipides. Ce complément contient donc un peu plus de phospholipides que l'autre mais plutôt sous forme d'oméga-3 EPA phospholipides (68 %). Soit plus de 456 mg d'oméga-3 EPA phospholipides ; mais avec seulement 168 mg d'oméga-3 DHA phospholipides. La phase grasse est constituée d'huile de krill selon un rapport DHA /EPA de 8/17

Si l'on compare les deux extraits on s'aperçoit donc que la différence réside dans le rapport DHA /EPA phospholipides qui est inversé. Les personnes du groupe Pc DHA ont surtout pris des oméga-3 DHA Phospholipides tandis que celles du groupe Pc EPA ont surtout des oméga-3 EPA Phospholipides.

La méthodologie pour évaluer le psoriasis a consisté à utiliser les deux critères majeurs de quantification du psoriasis à savoir le score PASI (*Psoriasis Area Severity Index)* et le score DLQI (Dermatology Life Quality Index).

Chacun des participants a pris le complément alimentaire qui lui était attribué de manière aléatoire par randomisation pendant 4 mois. L'évaluation de l'évolution du psoriasis s'est faite par photographie, par questionnaire d'autoévaluation du score PASI et du DLQI. Le PASI est le score de sévérité le plus utilisé. Mais comme il n'est pas très adapté pour l'évaluation de faibles surfaces cutanées atteintes, il a été associé au questionnaire DLQI qui évalue le ressenti du patient par d'autres signes qui permettent de quantifier le retentissement du psoriasis dans la vie quotidienne.

Les résultats de l'étude N1 ont montré que le groupe Pc DHA a atteint au bout de 4 mois en moyenne le PASI 45, c'est-à-dire une réduction moyenne de 45 % du psoriasis, passant d'un PASI moyen de 11,17 à un PASI moyen de 6,05. L e DLQI a permis d'observer également une réduction de 18 % du ressenti passant pour le groupe Pc DHA d'un DLQI moyen de 11,18 à un DLQI moyen de 9.56. (figure 1 ci-dessous)

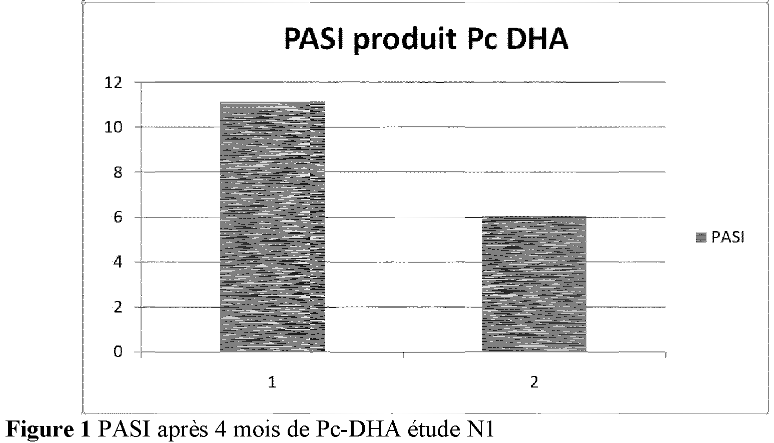

Quant au groupe Pc EPA la moitié des personnes ont stoppé la prise du complément lorsqu'ils ont observé une aggravation et il leur a été alors conseillé au bout de 2 mois de prendre le complément Pc DHA. Seuls ceux ayant un psoriasis minime ont continué les 4 mois : à cette date le PASI moyen des personnes sous Pc EPA a augmenté de 57 % passant de 1.42 à 1.86. Leur DLQI est resté stable.

La présente étude a donc permis de se rendre compte que toutes les lécithines marines ou tous les composés phospholipidiques riches en oméga-3 n'ont pas le même effet sur le psoriasis. Il apparaît clairement que ce sont les phospholipides riches en oméga-3 DHA qui sont véritablement actifs.

### Étude numéro 2 : N2

Une autre étude nommée étude N2 a été menée parallèlement à l'étude N1 et a regroupé 20 personnes avec tous les types de psoriasis : en plaques, gouttes, palmo-plantaires, du cuir chevelu, inversé. Tous les participants ont pris le protocole Pc DHA. La prise du complément a été plus longue, permettant d'obtenir de meilleurs résultats. Apres 6 mois les résultats sont en effet excellents car en moyenne le PASI 75 est atteint: le PASI 75 qui correspond à 75 % d'amélioration du psoriasis est considéré comme validant l'efficacité d'un soin du psoriasis : ainsi, dans l'étude N2 on obtient au bout de six mois en moyenne une baisse du PASI de 6,92 à 0,64, soit une amélioration de 90% du PASI. Le DLQI est également amélioré de 83 % passantde 9,6 à 1.6. (figure 2 ci-dessous)

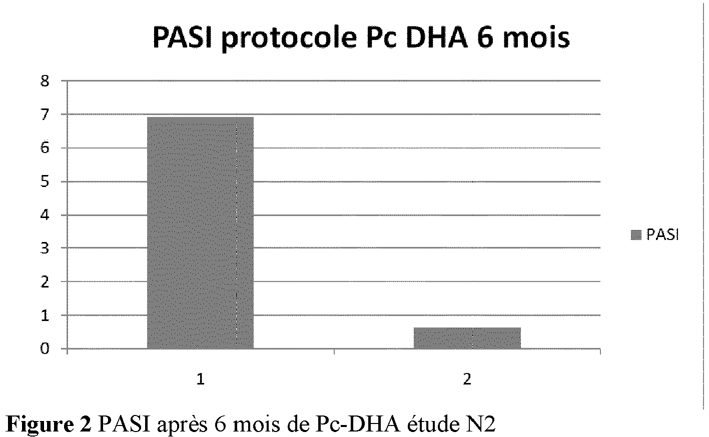

### Quelques cas significatifs

A Titre d'exemple voici la description de quelques cas parmi les plus caractéristiques de l'étude : d'abord 4 personnes ayant pris le composé Pc-DHA, et deux personnes avec le composé Pc-EPA.

### Exemple de 4 personnes ayant du psoriasis et prenant Le composé Pc-DHA

Mm B. Zoh est une femme de 44 ans. Elle présente un psoriasis en plaques et en gouttes qui couvre entre 10 à 20% de la surface corporelle. Les ongles sont également touchés. Le PASI est de 29,58. Elle remarque des rougeurs modérées sur sa tête et bras. Par contre, les rougeurs de ses jambes sont très sévères. Le psoriasis l'affecte psychologiquement avec une valeur de DLQI de 14. Elle prend l'extrait Pc-DHA.

Après 2 mois le PASI est passé de 29,58 à 5,22. Le psoriasis reste néanmoins sévère sur les jambes en termes de rougeur, d'épaisseur et de desquamation et notamment parce qu'elle se gratte. Malgré le changement important du niveau PASI, le niveau psychologique n'a pas beaucoup changé, avec un niveau DLQI de 13.

Cette personne va ensuite continuer la prise de Pc DHA. Après une année, elle n'a plus de psoriasis sur le tronc. La valeur du PASI est de 6,6 et le DLQI montre une nette amélioration de l'aspect psychologique avec une valeur descendue à 6.

Mr J. Ghr, un homme de 73 ans, souffre de psoriasis en plaques, en gouttes et pustuleux sur le cuir chevelu, le visage, le tronc, les membres, les plis, la région génitale et les ongles. Au début de l'étude N2, il présentait des érythèmes, une induration et une desquamation légers sur la tête, modérés sur le tronc, sévères sur les bras et très sévères sur les jambes. L'impact psychologique (DLQI) était élevé, affectant considérablement sa vie quotidienne et ses relations. Après deux mois de prise de Pc-DHA, il montrait des améliorations sur les bras et les jambes, avec des symptômes modérés dans plusieurs zones. Psychologiquement, l'impact restait significatif mais avec une légère amélioration du prurit. Après cinq mois, le psoriasis continuait à s'améliorer, surtout sur la tête et le tronc, bien que les jambes et les bras présentent encore des symptômes sévères. Psychologiquement, il notait une amélioration de sa vie sociale et de la gestion des symptômes, tout en continuant à utiliser le Pc-DHA.

À la fin de l'étude N2, les symptômes sur la tête, le tronc et les bras étaient modérés, tandis que les jambes restaient sévèrement affectées. L'impact psychologique (DLQI) montrait une amélioration significative. Il a alors décidé de continuer de lui-même la prise de Pc-DHA.

Lors de la dernière évaluation, il ne présentait plus que des symptômes légers sur la tête et le tronc, et modérés sur les bras et les jambes. L'impact psychologique s'était nettement amélioré, avec de légères gênes dans plusieurs aspects de sa vie. Il décrit une amélioration significative grâce au traitement, en particulier en ce qui concerne la réduction de la gravité des symptômes et de l'impact psychologique.

Mme J. STO, une femme de 36 ans qui souffre de psoriasis en gouttes et présent sur le cuir chevelu, le tronc, les bras et les jambes, sur toutes les parties de son corps. Son psoriasis en termes de desquamation, est sévère à très sévère sur toutes les parties. Cependant, elle ne remarque aucune rougeur ou épaississement sur aucune partie. Au début de l'étude elle a un PASI 8,9 et un DLQI 15. Elle a donc pris le produit Pc-DHA. Selon ses commentaires : « Au début je n'avais pas vu beaucoup de résultats ». Mais quand elle a commencé à se sentir mieux mentalement et ceci combiné avec la pris de Pc-DHA, elle a vu que les taches ont commencé à disparaître petit à petit et elles étaient de plus en plus fines. Trois mois après le début de l'étude son psoriasis était toujours de type en gouttes et persistait sur son cuir chevelu, son tronc et ses membres. Mais ses nouvelles valeurs PASI et DLQI étaient respectivement de 8,5 et 13.

Mme L. Kiel, une femme de 34 ans, a été suivie pour des symptômes de psoriasis sévères. Lors de la première évaluation, elle présentait des lésions sévères sur plusieurs zones de son corps, notamment le tronc, les membres et les plis cutanés. Les plaques étaient évaluées comme sévères, avec une gravité de 3 sur 3 pour ces zones. Les gouttes et les pustules étaient également sévères, avec un impact élevé sur sa qualité de vie. Son PASI initial était de 44,28. En ce qui concerne l'impact psychologique de la maladie, son DLQI est de 18. Elle a donc pris Pc-DHA et après 3 mois elle a noté une amélioration significative. Les lésions sévères avaient été considérablement réduites. Les symptômes avaient évolué vers des niveaux légers à modérés, avec des évaluations de 0 à 1 pour la gravité des plaques et des gouttes. Les zones affectées incluaient le tronc et les membres, où les lésions étaient désormais moins graves. L'impact psychologique avait également diminué, indiquant une amélioration notable dans sa qualité de vie. A la fin de l'étude son PASI n'est plus que de 0,66.

### Exemple de 5 personnes avec du psoriasis prenant le composé Pc-EPA

J. CEL, une femme de 80 ans, a été suivie pour des symptômes de psoriasis. Lors de la première évaluation, ses symptômes étaient sévères. Les lésions, particulièrement sur le tronc et les membres, étaient évaluées comme sévères, avec un impact psychologique élevé qui affectait considérablement sa qualité de vie. Lors de l'évaluation après 3 mois de Pc-EPA, il est apparu clairement que le composé n'avait pas produit les effets escomptés. Les lésions sur la tête et le cou restaient sévères, avec des érythèmes, indurations et desquamations toujours graves, affectant une surface corporelle importante de 30% à 49%. Cette gravité a eu un impact profond sur la qualité de vie de cette dame, comme l'indiquaient les scores élevés sur le DLQI. Les résultats montrent que le traitement n'a pas été efficace pour réduire significativement la gravité des symptômes de psoriasis. Le composé Pc EPA n'a pas réussi à améliorer de manière substantielle son état ou sa qualité de vie.

N.Bart, un homme de 32 ans, avait avant de commencer l'étude un psoriasis en goutte et en plaque avec un PASI de 15,8 et prenant déjà Pc DHA avant l'étude. Et au début de l'étude Son PASI était passé à 0.45. Dans la randomisation, il a reçu la Pc EPA et au bout de 3 mois le psoriasis a recommencé à s'aggraver avec un PASI à 2.21 mais surtout il a noté un rebond avec une poussée du psoriasis sur les ongles (8 ongles touchés sur les mains), 4 ongles pour les pieds. Il a présenté des douleurs musculaires et articulaires de type rhumatisme psoriasique pendant la prise de Pc EPA avec un légère augmentation des inflammations par gouttes, réparties sur tout le corps. Il za du reprendre de lui-même le protocole Pc DHA

C. Her. une femme de 33 ans avec un PASI modéré de 0.6 mais un DLQI de 12 n'a noté aucune amélioration après 3 mois de Pc EPA : PASI 0.54 et DLQI identique de 12.

P. Moru. Un homme de 59 ans sous Pc EPA a une augmentation des plaques avec un PASI qui passe de 1.26 à 2.46. et du DLQI aggravé de 8 à 9.

I. Que. Une femme de 54 ans son psoriasis après 3 mois de Pc-EPA s'est aggravé. Elle note : « Mon pso a flambé considérablement, et il me gratte énormément. »

A la fin de ces deux études il a ici été permis de conclure que seule la composition de phospholipides riches en oméga-3 DHA (Pc-DHA) est véritablement active dans le psoriasis. C'est donc ce composé qui fait l'objet de la présente invention.

## Revendications

1. Utilisation d'un phospholipide estérifié par des acides gras polyinsaturés de type oméga-3 DHA ou d'un extrait riche en oméga-3 DHA sous forme de phospholipides pour la fabrication d'un médicament administré par voie orale, rectale ou lymphatique directe,_utile dans la prévention et le traitement des états provoqués ou se manifestant par des formations squameuses ou kératosiques anormales du derme ou pour la préparation d'un complément alimentaire destiné au maintien d'une peau normale.
- L'extrait riche en oméga-3 DHA sous forme de phospholipides comprend les phospholipides suivants, en poids rapporté au poids total des phospholipides :
- de 30% à 85% de phosphatidylcholine, de préférence 50%,
- de 10% à 30% de phosphatidylinositol, de préférence 25%,
- de 5% à 30% de phosphatidyléthanolamine, de préférence 10%,
- de 5% à 20% de phosphatidylsérine, de préférence 10%, et
- de 5% à 20% de sphingomyéline, de préférence 5%.
- Les phospholipides sont estérifiés par un acide gras polyinsaturé du type oméga3-DHA.
- Le phospholipide estérifié par des acides gras polyinsaturés de type oméga-3 DHA peut être chacun des phospholipides de l'extrait mais préférentiellement la phosphatidylcholine.

2. Utilisation selon la revendication 1 ***caractérisée en ce que*** lesdits états sont choisis parmi le psoriasis habituel, en gouttes, nummulaires, en plaques, le psoriasis érythrodermique, le psoriasis arthropathique, le psoriasis pustuleux, le psoriasis de l'enfant, le psoriasis du cuir chevelu, la pulpite psoriasique, le psoriasis des ongles ou des muqueuses, le parapsoriasis, l'eczéma, les dermatoses aiguës ou chroniques.

3. Utilisation selon la revendication 1 ou 2 ***caractérisée en ce que*** ledit extrait soit riche en acides gras polyinsaturés oméga-3 DHA phospholipides dans une quantité de 10% à 50% en poids, de préférence 30% en poids, dans une phase grasse.

4. Utilisation selon l'une des revendications 1 à 3 ***caractérisée en ce que*** l'extrait riche en oméga-3 DHA phospholipides comprend les phospholipides suivants, en poids rapporté au poids total des phospholipides :
- de 30% à 85% de phosphatidylcholine, de préférence 50%,
- de 10% à 30% de phosphatidylinositol, de préférence 25%,
- de 5% à 30% de phosphatidyléthanolamine, de préférence 10%,
- de 5% à 20% de phosphatidylsérine, de préférence 10%, et
- de 5% à 20% de sphingomyéline, de préférence 5%.

5. Utilisation selon l'une des revendications 1 à 4 ***caractérisée en ce que*** l'extrait riche en oméga-3 DHA phospholipides comprend les phospholipides suivants, en poids rapporté au poids total des phospholipides de l'extrait :
- 50% de phosphatidylcholine,
- 25% de phosphatidylinositol,
- 10% de phosphatidyléthanolamine,
- 10% de phosphatidylsérine, et
- 5% de sphingomyéline.

6. Utilisation selon l'une des revendications 4 ou 5 ***caractérisée en ce que la majorité*** desdits phospholipides sont estérifiés par d'un acide gras polyinsaturés du type oméga3-DHA.

7. Utilisation selon la revendication 6 ***caractérisée en ce que dans*** ledit extrait riche en oméga-3 DHA phospholipides, les phospholipides sont estérifiés par, en poids rapporté au poids total desdits acides gras :
- l'acide docosahexanoïque à raison de 30% à 85%,
- l'acide eicosapentanoïque à raison de 5% à 35%,
- l'acide docosapentanoïque à raison de 0,5% à 5%,
- l'acide gras de type alkyl-glycérol à raison de 5% à 30%.

8. Utilisation selon l'une des revendications 6 ou 7 ***caractérisée en ce que*** ledit extrait riche en oméga-3 DHA phospholipides, les phospholipides sont estérifiés par, en poids rapporté au poids total desdits acides gras :
- l'acide docosahexanoïque à raison d'au moins 50 %,
- l'acide eicosapentanoïque à raison d'au moins 16 %,
- l'acide docosapentanoïque à raison d'au moins 0,5%,
- l'acide gras de type alkyl-glycérol à raison d'au moins 5%.

9. Utilisation selon l'une des revendications 1 à 8 ***caractérisée en ce que*** le phospholipide estérifié par des acides gras polyinsaturés de type oméga-3 DHA ou l'extrait riche en oméga-3 DHA phospholipides provient d'un organisme animal ou végétal choisi de préférence parmi les poissons, les crevettes, le krill, le zooplancton, les algues, le phytoplancton, ou extrait d' œuf d'origine animale ou bien encore d'un mélange de ceux-ci.

10. Utilisation selon l'une des revendications 1 à 9 ***caractérisée en ce que*** le phospholipide estérifié par des acides gras polyinsaturés de type oméga-3 DHA ou l'extrait riche en oméga-3 DHA phospholipides, ladite phase grasse est une huile de poisson, une huile extraite d'algue de préférence une huile riche en oméga-3 DHA ou d'une lécithine extraite d'un organisme vivant.

11. Utilisation selon l'une des revendications 1 à 3 ***caractérisée en ce que*** le ou les phospholipides estérifiés par des acides gras polyinsaturés de type oméga-3 DHA sont obtenus par synthèse enzymatique de phospholipides structurés enrichis en DHA.

12. Utilisation selon l'une des revendications 1 à 11 ***caractérisée en ce que*** le ou les phospholipides estérifiés par des acides gras polyinsaturés de type oméga-3 DHA sont administrés par voie orale et sous la forme de complément alimentaire. De sorte que le produit puisse être utilisé selon une posologie permettant l'apport d'une dose efficace, se situant entre 400 mg et 1000 mg d'oméga-3 DHA phospholipides par jour. La forme orale est préférée, car la prise induit très peu de contrainte, ce qui permet une bonne tolérance des patients pour des cures d'une durée de plusieurs mois.

13. Composition thérapeutique d'un médicament administré par voie orale, rectale ou lymphatique directe,_utile dans la prévention et le traitement des états provoqués ou se manifestant par des formations squameuses ou kératosiques anormales du derme, contenant un phospholipide estérifié par des acides gras polyinsaturés de type oméga-3 DHA ou un extrait riche en oméga-3 DHA sous forme de phospholipides à titre de principe actif.
- L'extrait riche en oméga-3 DHA sous forme de phospholipides comprend les phospholipides suivants, en poids rapporté au poids total des phospholipides :
- de 30% à 85% de phosphatidylcholine, de préférence 50%,
- de 10% à 30% de phosphatidylinositol, de préférence 25%,
- de 5% à 30% de phosphatidyléthanolamine, de préférence 10%,
- de 5% à 20% de phosphatidylsérine, de préférence 10%, et
- de 5% à 20% de sphingomyéline, de préférence 5%.
- Les phospholipides étant estérifiés par un acide gras polyinsaturé du type oméga3-DHA.
- Le phospholipide estérifié par des acides gras polyinsaturés de type oméga-3 DHA peut être chacun des phospholipides de l'extrait mais préférentiellement la phosphatidylcholine.

14. Composition thérapeutique selon la revendication 13, dans laquelle l'extrait riche en oméga-3 DHA phospholipides, comprend les phospholipides suivants, en poids rapporté au poids total des phospholipides :
- de 30% à 85% de phosphatidylcholine, de préférence 50%,
- de 10% à 30% de phosphatidylinositol, de préférence 25%,
- de 5% à 30% de phosphatidyléthanolamine, de préférence 10%,
- de 5% à 20% de phosphatidylsérine, de préférence 10%, et
- de 5% à 20% de sphingomyéline, de préférence 5%.

15. Composition thérapeutique selon les revendications 13 ou 14, dans laquelle l'extrait riche en oméga-3 DHA phospholipides, comprend les phospholipides suivants, en poids rapporté au poids total des phospholipides de la lécithine :
- 50% de phosphatidylcholine,
- 25% de phosphatidylinositol,
- 10% de phosphatidyléthanolamine,
- 10% de phosphatidylsérine, et
- 5% de sphingomyéline.

16. Composition thérapeutique selon l'une des revendications 14 ou 15 dans laquelle lesdits phospholipides sont estérifiés par des acides gras polyinsaturés du type oméga3 DHA.

17. Composition thérapeutique selon l'une des revendications 14 à 16 dans laquelle lesdits phospholipides sont estérifiés par, en poids rapporté au poids total desdits acides gras:
- l'acide docosahexanoïque à raison de 30% à 85%,
- l'acide eicosapentanoïque à raison de 5% à 35%,
- l'acide docosapentanoïque à raison de 0,5% à 5%,
- l'acide gras de type alkyl-glycérol à raison de 5% à 30%.

18. Composition thérapeutique selon l'une des revendications 14 à 17 dans laquelle lesdits phospholipides sont estérifiés par, en poids rapporté au poids total desdits acides gras:
- l'acide docosahexanoïque à raison d'au moins 50%,
- l'acide eicosapentanoïque à raison d'au moins 14%,
- l'acide docosapentanoïque à raison d'au moins 0,5%,
- l'acide gras de type alkyl-glycérol à raison d'au moins 5%.

19. Composition thérapeutique selon l'une des revendications 13 à 18 dans laquelle le phospholipide estérifié par des acides gras polyinsaturés de type oméga-3 DHA ou l'extrait riche en oméga-3 DHA phospholipides est un extrait animal ou végétal choisi de préférence parmi les poissons, les crevettes, le krill, le zooplancton, les algues, le phytoplancton, ou d'œuf d'origine animale ou d'une mélange de ceux-ci.

20. Composition thérapeutique selon l'une des revendications 13 à 18, dans laquelle dans l'oméga-3 DHA phospholipide est apporté sous forme d'extrait dans une phase grasse, ladite phase grasse est une huile de poisson, une huile extraite d'algue de préférence une huile riche en oméga-3 DHA ou une lécithine extraite d'un organisme vivant.

21. Composition thérapeutique selon l'une des revendications 13 à 18 ***caractérisée en ce que*** le ou les phospholipides estérifiés par des acides gras polyinsaturés de type oméga-3 DHA sont obtenus par synthèse enzymatique de phospholipides structuré riche en DHA.
